# EUROPEAN PATENT APPLICATION

(11) **EP 1 013 268 A1**
(43) Date of publication of application: **28.06.2000**
(21) Application number: 99957621.8
(22) Date of filing: 29.06.1999
(51) Int. Cl.: A61K 9/107, A61K 9/127

(54) **COMPOSITIONS CONTAINING LIPOSOMES AND/OR EMULSIONS AND PROCESS FOR THE PREPARATION THEREOF**

(30) Priority: 30.06.1998 JP 18400098
(71) Applicant: ROHTO PHARMACEUTICAL CO., LTD., Osaka-shi, Osaka 544-8666 (JP)
(72) Inventor: FUKUI, Masaru, Rohto Pharmaceutical Co., Ltd, Osaka-shi, Osaka 544-8666 (JP); MORIOKA, Shigeo, Rohto Pharmaceutical Co., Ltd, Osaka-shi, Osaka 544-8666 (JP)
(74) Representative: Stuart, Ian Alexander
(86) International application number: JP9903467
(87) International publication number: WO0000178

(57) **Abstract**

The present invention provides a composition containing liposome and/or emulsion, characterized in that it is prepared by colliding the following two solutions at the time of use: (1) an oil-phase containing a surfactant; and (2) an aqueous phase, wherein at least one phase contains an active ingredient.

## Description

### TECHNICAL FIELD

The present invention relates to a liposome- and/or emulsion-containing composition which is prepared at the time of use and is useful in various fields including cosmetics and medicines, and a method for the preparation thereof.

### BACKGROUND ART

When a hydrophilic or lipophilic active ingredient is applied to skin or mucosa, it is generally used as a composition In the form of an ointment or a solution. An ointment is classified into lipophilic, hydrophilic or emulsified ointment depending on the kind of the base. It also requires various additives such as an emulsifier, a preservative, and the like, in addition to the base to retain the stability of the preparations. These substances may often be irritant to skin and mucosa and cause a hypersensitive reaction. Further, an ointment is generally used for a long term after opening and, therefore, should be free from substances susceptible to chemical or physical change during storage. Accordingly, the freedom in the formula design is necessarily restricted.

On the other hand, liquid compositions containing liposome or emulsion as a carrier for an active ingredient show a broad usage in the field of cosmetics and medicine due to the possibility of improving the bioavailability and/or reducing the cutaneous or mucosal irritation. However, it is quite difficult to maintain a liposomal or an emulsion composition in well-dispersed state for a long period because such a composition exists in a meta-stable system". There have been many approaches towards the prevention of aggregation or fusion of liposome or emulsion, which comprises, for example, using a chemically modified phospholipid or a combination of phospholipid and charged substance, or coating particles with a polyhydric alcohol or sugar (FRAGRANCE JOURNAL, 1996-4, pp.85-93: Pharmacia, 21,1229 (1985)). Lyophilization process has also been proposed as a means for preserving liposomal or emulsion composition for a long period (Int. J. Pharm, 22, 299 (1984)). However, it is difficult by any of known methods to maintain a composition for a long period in a state similar to that of immediately after the preparation, wherein liposome and/or emulsion particles disperse homogeneously in a medium and an active ingredient is present stably. For this reason, the reduction of quality has been unavoidable. Further, the stability of a lyophilized preparation greatly damaged by the method of reconstitution with water, the reconstitution process, therefore, requires quite delicate conditions and can hardly be conducted by ordinary person in ease. Accordingly, additives such as dispersing co-agent are essential to stabilize liposome or emulsion, which may often cause irritable or hypersensitive reaction. Further, an interaction among active ingredients, or among an active ingredient(s) and one or more liposome- or emulsion-forming substances can take place during storage, and the freedom of formula design could be restricted.

It has been described in JP04-281835A a method for preparing an emulsion composition stable during a long-term storage, which comprises colliding emulsion materials by allowing them to pass through a nozzle to yield fine particles and further adding dispersing co-agent thereto, which emulsion materials have been prepared using an ordinary dispersing co-agent. This method, however, cannot solve the problem of the restricted freedom of formula design and still requires a considerable amount of additives to ensure the stability during storage. On the other hand, JP60-894 14A disclosed a method for preparing an aerosol spray containing liposome, which comprises mixing two separate components under pressure and allowing the resultant mixture to pass through a nozzle under pressure. This method is intended to prepare an aerosol composition of liposome including non-lipophilic active ingredient and is not applicable to the preparation of compositions containing a lipophilic active ingredient.

### DISCLOSURE OF THE INVENTION

Accordingly, there has been a demand for the development of a liposome- and/or emulsion-containing composition of much safer and excellent dispersibility, which is capable of containing any kinds of active ingredients regardless of lipophilicity, hydrophilicity or incompatibility, which can be used with a constant quality while avoiding the problems during a long-term storage such as physical changes due to aggregation or fusion of particles, or chemical interaction between ingredients, and which requires no or only minimum amount of an additive(s) such as dispersing co-agent.

The present invention solves the above-mentioned problems and provides a liposome- and/or emulsion-containing composition, characterized in that it is prepared by colliding the following two solutions at the time of use:
(1) an oil-phase containing a surfactant; and
(2) an aqueous phase, wherein at least one phase contains an active ingredient and a method for preparing the said composition.

In one preferred embodiment, a hydrophilic and a lipophilic active ingredients are each dissolved into the aqueous and oil-phases, respectively, and the two phases are collided to provide a composition containing liposome and emulsion as a carrier for the hydrophilic and the lipophilic active ingredients, respectively.

In another preferred embodiment, an aqueous phase and an oil-phase containing a lipophilic active ingredient and a surfactant are collided at the time of use to provide a composition containing emulsion as a carrier for the lipophilic active ingredient.

Although a composition containing only a lipophilic active ingredient above can contain liposome, it is preferred that the content of liposome is as little as possible. It is more preferable that the emulsion composition is free from liposome.

### BRIEF EXPLANATION OF DRAWINGS

Fig. 1 shows the distribution of particle diameter of liposome and emulsion in the composition containing liposome and O/W emulsion prepared In Example 2.
Fig. 2 shows the distribution of particle diameter of emulsion in the composition containing emulsion prepared in Example 4.
Fig. 3 shows the distribution of diameter of particles in the composition containing liposome and emulsion prepared in Example 6.
Fig. 4 shows the result of an *in vitro* cutaneous permeation test conducted using an emulsion composition of the present invention containing a mixture of drugs (Example 4), an ointment (Comparative Example 2), and a propylene glycol solution containing the same mixture of drugs as the composition of Example 4 (Comparative Example 3).
Fig. 5 shows the result of an *in vitro* cutaneous permeation test conducted using a liposome- and emulsion-containing composition of the present invention (Example 6), and a solution containing 1% ascorbic acid phosphate magnesium salt (Comparative Example 4).
Fig. 6 is a graph depicting the cutaneous permeation amount of ascorbic acid and ascorbic acid phosphate magnesium salt after 24 hours in the test shown in Fig. 5.
Fig. 7 is a graph depicting the amount of ascorbic acid and ascorbic acid phosphate magnesium salt retained intradermally after 24 hours in the test shown in Fig. 5.

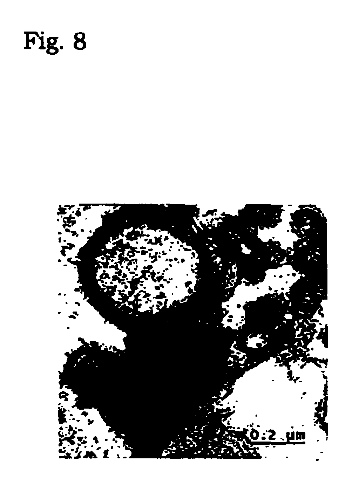

Fig. 8 is a photograph of a liposome- and emulsion-containing composition prepared in Example 6 observed under a transmission electron microscope.

### BEST MODE FOR CARRYING OUT THE INVENTION

In the present specification, the terms "liposome and "emulsion" are used in the same meaning as known in the art. Thus, the term "liposome" refers to a lipid vesicle surrounded with a lipid bilayer, which can be a carrier for a hydrophilic substance or protein. The term "emulsion" refers to a milky dispersed system wherein the disperse-phase and -medium are immiscible. The emulsion can be classified into W/O type and O/W type by the kind of disperse-phase and -medium. Recently, a method for preparing W/O/W type or O/W/O type emulsion has been established, which comprises further dispersing W/O type or O/W type emulsion in a disperse medium. In carrying out the present invention, either O/W type or W/O/W type emulsion in which lipid particles are dispersed into an aqueous solvent is preferred, and the O/W type emulsion is more preferred. As herein used, the term "emulsion" refers not only to an emulsion composition but also to emulsion particles.

The composition of the present invention containing liposome and/or emulsion can be prepared by colliding two solutions under the conditions suited for the production of liposome and/or emulsion at the time of use, which solutions have been previously prepared and kept separately. In the method, one of the solutions is an oil-phase which contains natural and/or synthetic surfactant which serves as a liposomal membrane- or emulsion-forming substance, the other is an aqueous phase, and at least one of two solutions contains an active ingredient(s).

The collision of two solutions can be conducted by any methods subject that liposome and/or emulsion is formed, for example, preferably by bringing two solutions into collision using a spray mechanism of a nebulizer of handy-size or by shaking appropriate portions of the solutions together. The method employing a spray mechanism is preferred since it is easy to operate and yields a product stably and reproducibly regardless of the skill or power of the person who deals with. Generally, a nebulizer consists of a spray mechanism and a container for a liquid. The container is provided with a dip tube and a valve inside and at the opening thereof, respectively, such that a pressured liquid in the container reaches the valve via the dip tube and then the spray mechanism, and finally jets out. Such a nebulizer is well-known in various fields including medical field, and any one of them can be used for carrying out the present invention.

The present invention can be carried out by the use of a nebulizer or a spray apparatus having two containers/chambers each separately storing oil- or aqueous phase and a spray mechanism wherein the two solutions are collided. In this case, the spray mechanism can consist of a mixing part into which two solutions are introduced from each container/chamber and collide under pressure, and optionally a spray part from which the resultant composition containing emulsion and/or liposome is sprayed. At the mixing part, ends of two tubes each being connected to a valve (which is connected to a dip tube) of each container meet together and two solutions are mixed by collision. The mixing part can be a "mixing nozzle". A composition containing minute liposome and/or emulsion can be formed when oil- and aqueous phases are charged in each chamber of a nebulizer equipped with the above-mentioned spray mechanism and then pressure is applied so that two solutions can collide at the mixing part. The above-mentioned embodiment is shown for illustrative purpose and the present invention is not restricted to any specific structure or mechanism of nebulizer.

The preferred mean particle diameter of liposome or emulsion particles varies depending on the intended use. However, it is preferably 1 µ or less, more preferably 500 nm or less, and especially preferably 200 nm or less, because particles of smaller size are more desirable for attaining the efficient absorption through dermis or mucous membrane. Although there is no specific lower limit of particle diameter, many of the particles tend to form micelle under particle diameter of 20nm, which can include little hydrophilic substance and extremely little lipophilic substance. Accordingly, the lower limit of mean particle diameter is preferably 20 nm in general, considering the structural stability of liposome and emulsion particles.

The particle diameter can be adjusted by selecting appropriate conditions such as the amount of surfactant, the components of the solvent and the pressure at the time of collision. Such conditions may be selected from those known in the art.

In using a nebulizer, the collision pressure can be adjusted by regulating appropriately the internal pressure of a container, or the type (shape) of nozzle or valve.

The term "spray mechanism" used herein refers to a series of mechanisms consisting of a mixing part for collision of two solutions and a spray part for spray. The spray part may be omitted in some cases.

The term "nozzle" generally refers to a narrow opening through which a pressurized liquid jets out at high speed. The term "mixing nozzle", when used as a means to prepare a composition of the present invention from two solutions by collision, refers to a device where two pressurized liquids flow into, collide to form a liposome-and/or emulsion-containing mixture, and jet out, or a part thereof. The particle size or the mixing rate of two liquids can be adjusted by changing the diameter and/or the shape of the mixing nozzle, which can be done by known methods.

The ingredients of a composition containing liposome and/or emulsion of the present invention is hereinafter explained by way of examples.

An oil-phase containing natural and/or synthetic surfactant serves as a material for the formation of the membrane of liposome or emulsion particles. The term "surfactant", as used herein, refers to a substance with surface-active action. Examples of surfactant include substances with relatively weak surface activating activity such as phospholipid, bile acid, and the like, as well as those commonly known as surfactants.

Examples of "natural surfactant" include a phospholipid such as yolk lecithin, soybean lecithin, phosphatidyl choline, phosphatidyl ethanolamine, phosphatidyl serine, phosphatidyl inositol, sphingomyelin, and the like; a bile acid such as cholic acid, dehydrocholic acid, deoxycholic acid, and the like; a cholesterol derivative such as cholesterol, cholesteryl palmitate, cholesteryl myristate, cholesteryl stearate, and salts thereof, and the like.

Examples of "synthetic surfactant" include non-ionic, anionic, cationic and ampholytic surfactants and a synthetic phospholipid which are commonly used in the art.

Preferable surfactants are natural ones and phospholipids are especially preferred.

The content of a surfactant varies depending on the amount of other constitutions, especially the lipid ingredient, in the oil-phase in relation to the aqueous medium or to the ratio of liposome and emulsion particles to be contained in the composition. However, it is generally preferred that the oil-phase contains a surfactant sufficient to give a mixture containing surfactant at concentration ranging from 0.01 to 25 w/w% when combined with the aqueous phase. For the preparation of a composition containing hydrophilic and lipophilic active ingredients, that is, a composition containing both liposome and emulsion, the ratio of a surfactant to lipophilic ingredients is preferably between 1 : 5 and 1 : 50. For the preparation of emulsion composition free from liposome, the ratio of a surfactant to oily substances can be between 1 : 0.01 and 1 : 2.0, preferably, between 1 : 0.1 and 1 : 1.0. The present invention, however, is not limited to these ratios.

One, two, or more surfactants can be selected and used depending on the nature of an active ingredient(s) to be included.

A solvent in the oil-phase can be selected from polyhydric alcohols such as glycerin, ethylene glycol, diethylene glycol, triethylene glycol, propylene glycol, dipropylene glycol, butylene glycol, macrogol, etc.; alcohols such as methanol, ethanol, propanol, etc.; fatty acid esters of glycerin such as lauric acid monoglyceride, stearic acid triglyceride, etc.; fatty acid esters of sucrose such as sucrose stearate, sucrose palmitate, sucrose oleate, sucrose laurate, sucrose vehenate, etc.; natural plant oils such as soybean oil, sesame oil, rape seed oil, castor oil, etc.; and natural animal oils, and the like, or a mixture thereof.

An aqueous phase can be purified water optionally containing a hydrophilic active ingredient and other hydrophilic substances. Examples of other substances are additives including a solubilizing agent such as ethanol, a stabilizer such as EDTA, a hydrophilic absorption enhancer, a preservative, and the like.

The mixing ratio of oil-phase and aqueous phase is generally in the range of between 1 : 1 and 1 : 20, although it varies depending on the character of the intended composition.

A propellant may be included in each of the oil and aqueous phases to obtain a spray formulation containing liposome or emulsion by injecting the both phases. Suitable propellants can be selected from those generally used in the art and be in the form of liquid, gas, or liquefied gas, or the like. Examples of propellants usable in the present invention include dimethyl ether, carbon dioxide, nitrogen, air, LPG, and isopentane.

The pressure of the injection with a nozzle varies depending on the device used and is optional. However, it would be about 1 to 13 kg/cm² at temperature between 5 and 40 °C that required to obtain a composition containing emulsion and liposome particles of mean particle diameter suited for the present invention. The outlets of two chambers are each equipped with a valve unit. The mixing manner of two solutions can be changed by using a valve of different shape, for example, mixing at a fixed quantity, continuous mixing, or the like.

An active ingredient of any kinds can be included in the composition of the present invention using liposome or emulsion as a carrier. The active ingredient can be dissolved into oil and/or aqueous phase depending on the physical characteristics and/or the intended effect. Because the present composition has not been prepared in advance, that is, it is prepared by colliding two phases at the time of use, any one or more lipophilic and/or hydrophilic active ingredients can be dissolved in oil and/or aqueous phase. An active ingredient can be selected from any substances useful in cosmetics, drugs, or industry.

Because the composition of the present invention has improved characteristics, especially in the intradermal retention effect, it is especially preferred to contain a lipophilic or hydrophilic ingredient capable of activating skin cells.

Example of lipophilic ingredients include oil-soluble vitamins such as retinol, tocopherol acetate, retinol palmitate, retinol acetate, ascorbic acid palmitate, tocopherol, *dl*- α -tocopherol acetate, tocopherol succinate, panthenol, panthenyl ethyl ether, pyridoxine dicaprylate, etc.; hydrophobic peptides with elastase inhibitory activity, etc.; steroides (hormones) such as dexamethasone, dexamethasone acetate, hydrocortisone acetate, prednisolone acetate, etc.; antipruritics such as crotamiton, etc.; antihistaminics such as diphenhydramine, chlorphenylamine, or organic acid derivative thereof; local anesthetics such as lidocaine, dibucaine, etc.; various kinds of essential oil such as *l*-menthol, *dl*-camphor, peppermint oil, geraniol, *d*-borneol, eucalyptus oil, turpentine oil, etc.; anti-inflammatory agents such as glycyrrhezin, glycyrrhetic acid, glycyrrhizin, glycyrrhytic acid, etc.; emollients such as urea, vaseline, etc.; propylene glycol; plant extract; or extracts derived from galenicals.

Example of hydrophilic ingredients include polysaccharides such as sodium hyaluronate, sodium chondroitin sulfate, collagen, etc.; placenta extract, various kinds of amino acid; peptides or proteins, or a mixture thereof; glycoproteins; sugars; α -hydroxy acid; emollients such as sodium salt of pyrrolidone carboxylic acid, etc.; water-soluble vitamins such as riboflavin, thiamin, cyanocobalamin, ascorbic acid phosphate, ascorbic acid sulfate, ascorbic acid palmitate, pyridoxine hydrochloride, ascorbic acid, pantothenic acid and salts thereof, etc.; antibacterials such as berberine chloride, benzalkonium chloride, chlorhexidine hydrochloride, isopropylmethyl phenol, etc.; anti-inflammatory agents such as lysozyme chloride, dipotassium salt of glycyrrhytic acid, salicylic acid, methyl salicylate, salicylic acid glycol, guaiazulene, etc.; antifungals such as myconazol nitrate, econazol nitrate, oxyconazol nitrate, sulconazol nitrate, etc.; organic acids such as citric acid, succinic acid, lactic acid, and salts thereof, etc.

It is not essential to use a conventional dispersing co-agent for the stabilization of liposome and/or emulsion in a composition of the present invention, because these particles are formed at the time of use. However, a minimum amount of dispersing co-agent may optionally be used, if necessary. For example, a thickener such as carboxy methylcellulose, xanthan gum, or a higher alcohol such as wax can be used.

Although the composition of the present invention can be in any forms subject that it contains liposome and/or emulsion, it is preferably prepared by colliding a given amount of oil- and aqueous phases by spray at the time of use. Specifically, aerosol prepared by colliding oil and aqueous phases in a spray mechanism is preferably, since it can be sprayed in various forms such as mist, powder, foam or paste by arranging the diameter of nozzle hole or by changing the shape of valve or the kind of propellant. It is especially preferable that the composition of the present invention is sprayed in the form of mist. It is preferred that the mist is as fine as possible since the cutaneous permeability is improved as the particle size become smaller. The mean particle diameter of mist is preferably 200 µm or less, more preferably, 50 µm or less, and especially preferably 20 µm or less. The resultant aerosol is topically applicable as it is. It, however, is possible that a composition containing liposome and/or emulsion is prepared by collision in a container, and then removed from the container for application.

The composition of the present invention can mainly be formulated into endermics (percutaneous drugs), trans-mucosal drugs or inhalations, and is usable in a wide range of fields including medicinal drugs, quasi drugs, cosmetics, household articles and industrial articles. It is especially preferably used in the field of medical drugs, quasi drugs and cosmetics.

The following Examples are provided to further illustrate the present invention and are not to be construed as limiting the scope of the invention.

### Example 1 Liposome-containing Aerosol Composition

Purified egg yolk lecithin (5 g) was dissolved into 1:1 (v/v) mixture (95 g) of glycerin and ethanol as a solvent. A portion (66.7 g) of the solution and dimethyl ether (33.3 g) were charged in a pressure container to obtain the "first solution". In a separate pressure container were charged 5% solution (66.7 g) of ascorbic acid phosphate magnesium salt and dimethyl ether (33.3 g) to obtain the "second solution". Each container was fitted with a valve (φ 0.5 mm), connected to a mixing nozzle, and injected at room temperature to spray as mist of a single mixture solution. After spray, liposome of multi-lamella was formed in the mist.

### Example 2 Liposome- and Emulsion-containing Aerosol Composition

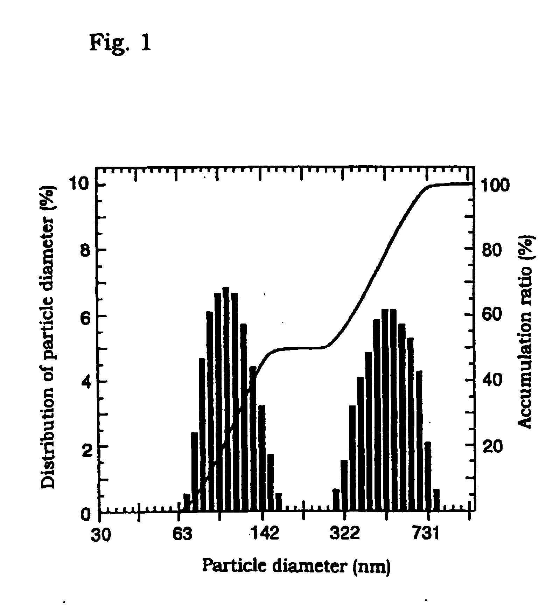

Purified egg yolk lecithin (10 g) and retinol palmitate (0.5 g) were dissolved in propylene glycol (93.5 g). The solution (90 ml) and carbon dioxide gas were charged in a pressure container at internal pressure of 10 kg/cm² to obtain the "first solution". In a separate pressure container were charged 1% aqueous solution (90 ml) of ascorbic acid phosphate magnesium salt and carbon dioxide gas at internal pressure of 10 kg/cm² to obtain the "second solution". Each container was fitted with a valve (φ 0.5 mm), connected to a mixing nozzle, and injected at room temperature to spray as mist of a single mixture solution. After spray, the particle size distribution of a mixture of liposome and O/W emulsion formed in the mist was measured with the laser light-scattering particle size analyzer (available from Otsuka Electronics Co., Ltd.) The distribution was expressed by the intensity ( 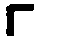 ) of scattering light. The result is shown in Fig. 1. Fig. 1 depicts the distribution of particle size calculated from the distribution of scattering intensity, wherein the horizontal axis represents the particle diameter (nm) and the left side vertical axis the particle size distribution (%). Prom the bar graph, it can be seen that the particle size distribution has two peaks with the mean particle diameter of 107 nm and 509nm, respectively. The right vertical axis represents the accumulation ratio (%) of particles.

### Example 3 Composition Containing Emulsion

Purified egg yolk lecithin (1 g) and retinol palmitate (0.5 g) were dissolved in propylene glycol (98.5 g). The solution (80 g) and dimethyl ether (20 g) were charged in a pressure container to obtain the "first solution". In a separate pressure container were charged physiological saline (80 g) and dimethyl ether (20 g) to obtain the "second solution". Each container for the first and second solutions was fitted with a valve (φ 0.5 mm), connected to a mixing nozzle, and injected at room temperature to spray as mist of a single mixture solution. After spray, the particle size distribution of emulsion formed in the mist was measured in a manner similar to that described in Example 2. In Comparative Example 1, the first solution was simply sprayed into physiological saline without mixing with a nozzle, and the resultant emulsion was subjected to the measurement of particle size distribution.

The results showed that the particle size distribution obtained in both Example 3 and Comparative Example 1 gave two peaks with the mean particle diameter of 57.7 nm and 403 nm (Example 3), and 288 nm and 10 µm (Comparative Example 1). It was revealed that the composition of Example 3 contains more minute emulsion than that of Comparative Example 1.

### Example 4 Composition Containing Emulsion

Purified egg yolk lecithin (5 g) and a mixture containing the following ingredients were dissolved in propylene glycol (83.2 g).

| Ingredients | Amount |
|---|---|
| Crotamiton | 5.0 g |
| Diphenhydramine | 1.0 g |
| *dl*- α -Tocopherol acetate | 0.5 g |
| Glycyrrhetic acid | 0.2 g |
| *l*-Menthol | 0.1 g |
| Urea | 5.0 g |
| Total | 11.8 g |

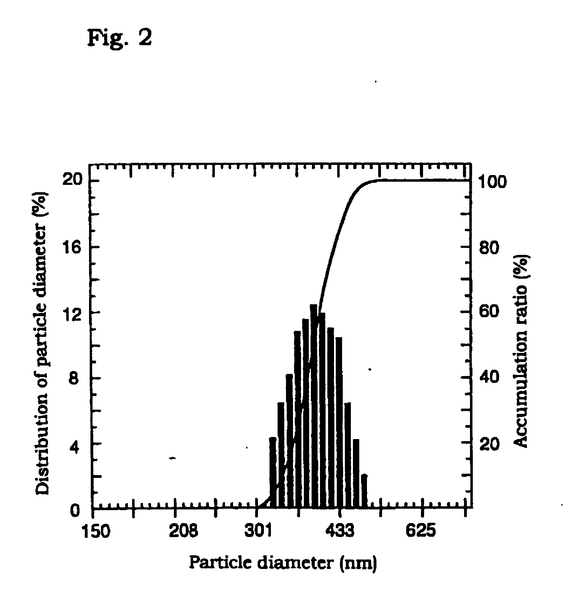

The solution (66.7 g) and dimethyl ether (33.3 g) were charged in a pressure container to obtain the "first solution". In a separate pressure container were charged physiological saline (66.7 g) and dimethyl ether (33.3 g) to obtain the "second solution". Each container for the first and second solutions was fitted with a valve (φ 0.5 mm), connected to a mixing nozzle, and injected at room temperature to spray as mist of a single mixture solution. After spray, the particle size distribution of emulsion formed in the mist was measured in a manner similar to that described in Example 2. The result is shown in Fig. 2. In the figure, the horizontal axis represents particle diameter (nm) and the left side vertical axis the particle size distribution (%). It can be seen from the particle size distribution shown in the bar graph that the mean particle diameter is 394 nm. The right vertical axis represents the accumulation ratio (%) of particles.

### Example 5 Composition Containing Emulsion

Purified egg yolk lecithin (5 g) and ingredient mixture described in Example 4 were dissolved in a mixture (83.2 g) of glycerin and ethanol (1:1, v/v). The solution (66.7 g) and dimethyl ether (33.3 g) were charged in a pressure container to obtain the "first solution". In a separate pressure container were charged physiological saline (66.7 g) and dimethyl ether (33.3 g) to obtain the "second solution". Each container for the first and second solutions was fitted with a valve (φ 0.5 mm), connected to a mixing nozzle, and injected at room temperature to spray as mist of a single mixture solution. After spray, the particle size distribution of emulsion formed in the mist was measured. The mean particle diameter was 557 nm.

### Example 6 Aerosol Composition Containing Liposome and Emulsion

Purified soybean lecithin (5 g) and a mixture containing the following ingredients were dissolved in a mixed solvent of dipropylene glycol and ethanol (85:15, w/w) (93.7 g).

| Ingredients | Amount |
|---|---|
| Tocopherol acetate | 0.3 g |
| Panthenol | 0.5 g |
| Pyridoxine dicaprylate | 0.5 g |

The solution (75 g) and dimethyl ether (25 g) were charged in a pressure container to obtain the "first solution". In a separate pressure container were charged an aqueous solution (75 g) containing the following ingredients and dimethyl ether (25 g) to obtain the "second solution".

| Ingredients | Amount |
|---|---|
| Ascorbic acid phosphate magnesium | 1% |
| β-Cyclodextrin | 1% |
| 1N Citric acid . | 15% |

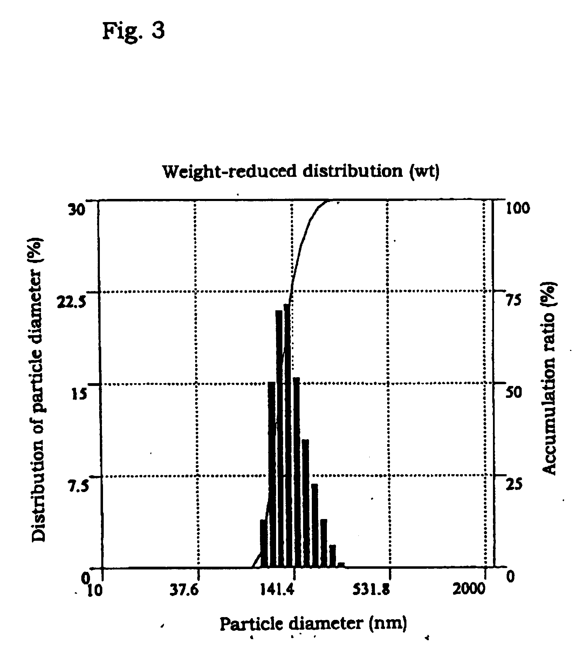

Each container for the first and the second solutions was fitted with a valve (φ 0.5 mm), connected to a mixing nozzle, and injected at room temperature to spray as mist of a single mixture solution. After spray, the particle size distribution of liposome and emulsion formed in the mist was measured in a manner similar to that described in Example 2. The result is shown in Fig. 3. It can be seen from the particle size distribution shown in the bar graph that the mean particle diameter is 132 nm. The right vertical axis represents the accumulation ratio (%) of particles.

### Example 7 Composition Containing Emulsion

Purified egg yolk lecithin (5 g) and a mixture containing the following ingredients were dissolved in a mixture of propylene glycol and ethanol (85:15, w/w) (57.5 g).

| Ingredients | Amount |
|---|---|
| Crotamiton | 10.0 g |
| Chlorpheniramine maleate | 2.0 g |
| Panthenol | 5.0 g |
| Glycyrrhitic acid | 0.4 g |
| *dl*-Camphor | 0.1 g |
| Urea | 20.0 g |

The solution (66.7 g) and dimethyl ether (33.3 g) were charged in a pressure container to obtain the "first solution". In a separate pressure container were charged physiological saline (66.7 g) and dimethyl ether (33.3 g) to obtain the "second solution". Each container for the first and the second solutions was fitted with a valve (φ 0.5 mm), connected to a mixing nozzle, and injected at room temperature to spray as mist of a single mixture solution. After spray, emulsion was formed in the mist.

### Example 8 Composition Containing Liposome and Emulsion

Purified soybean lecithin (5 g) and a mixture containing the following ingredients were dissolved in dipropylene glycol (88.5 g).

| Ingredients | Amount |
|---|---|
| Panthenol | 2.5 g |
| Tocopherol acetate | 1.5 g |
| Pyridoxine dicaprylate | 2.5 g |

The solution (75 g) and dimethyl ether (25 g) were charged in a pressure container to obtain the "first solution". In a separate pressure container were charged an aqueous solution (75 g) containing the following ingredients and dimethyl ether (25 g) to obtain the "second solution".

| Ingredients | Amount |
|---|---|
| Riboflavin | 1% |
| Ascorbic acid phosphate magnesium | 1% |
| α -cyclodextrin | 1% |
| 1N Citric acid | 15% |

The pH was adjusted to 7.0 by adding appropriate amount of sodium hydroxide. Each container for the first and second solutions was fitted with a valve (φ 0.5 mm), connected to a mixing nozzle, and injected at room temperature to spray as mist of a single mixture solution. After spray, the presence of emulsion in the mist was confirmed.

### Experimental Example 1 Cutaneous Permeation Test (1)

The cutaneous permeation test was carried out *in vitro* using abdominal skin samples removed from a male Wister rat (body weight of about 300 g).

The experiment was conducted in a thermostatic oven at 37 °C using a vertical cell (Franz-type) as evaluation cell. The cell was partitioned with the skin (5 cm square). PBS (pH 7.4, 45 ml) was charged in one part (the receptor side), and an emulsion composition of Example 4 (5 ml) in another (the donor side) as the test solution. The solution of receptor side was continuously stirred during the permeation test to avoid the occurrence of a density gradient of drug in the solution. At every point of time for sampling, 2 ml sample was collected from the solution of receptor side, and immediately supplemented with PBS of the same volume. The cutaneous permeability of each composition was evaluated using diphenhydramine as an index. The diphenhydramine level was measured by HPLC and evaluated according to the absolute calibration method.

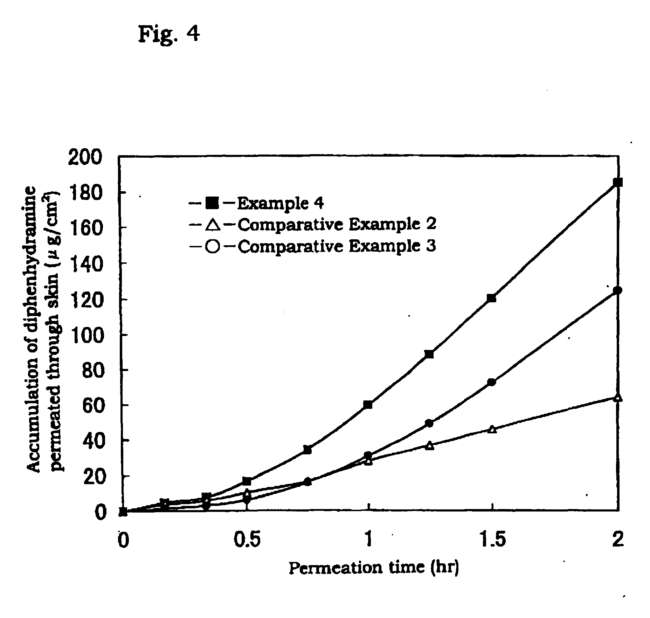

In Comparative Examples 2 and 3, the permeability was evaluated in a manner similar to that described above using as a sample, an ointment (2 g) containing the ingredient mixture described in Example 4, or a solution (2 g) of the same ingredient mixture in propylene glycol (88.2 g). The result is shown in Fig. 4.

It is apparent from the figure that the permeation rate is slow in Comparative Example 2 and that a long time lag is observed in Comparative Example 3. However, in the case of the composition of Example 4 of the present invention, no time lag is observed and the permeation rate is rapid.

### Experimental Example 2 Cutaneous Permeation Test (2)

The cutaneous permeation test was carried out *in vitro* using abdominal skin samples (3 cm square) removed from male HOS:HR-1 Hairless mice (6 to 8 weeks) .

The test was conducted using as an evaluation cell a horizontal cell (manufactured by VIDREX®) equipped with a jacket while circulating thermostatic water (37 °C) in the jacket. The cell was partitioned with the skin and PBS (pH 7.4, 5 ml) was charged in one part (the receptor side), and an emulsion composition of Example 6 (5 ml) in another (the donor side) as the test solution. The solutions of both sides were continuously stirred during the permeation test to avoid the occurrence of density gradient of drugs in the solutions. At every point of time for sampling, 200 µl sample was collected from the solution of receptor side and immediately supplemented with PBS of the same volume. The cutaneous permeability of each composition was evaluated on the basis of the amount of ascorbic acid and ascorbic acid phosphate magnesium salt permeated through the skin as an index. The determination of ascorbic acid and ascorbic acid phosphate magnesium salt was carried out using HPLC according to the absolute calibration curve method.

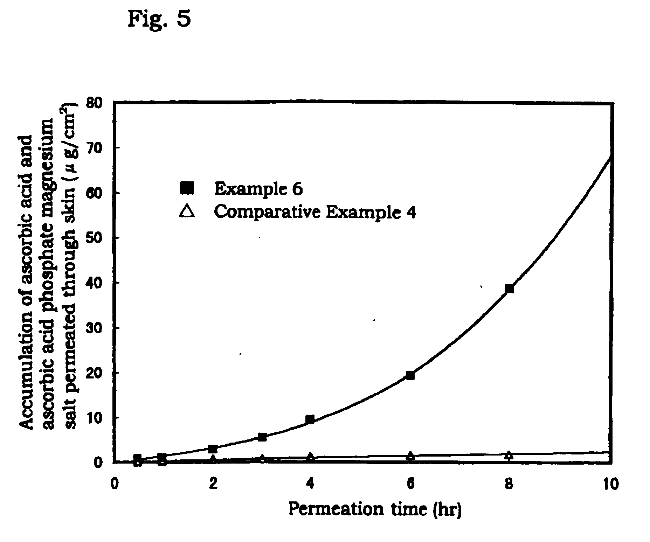

In Comparative Example 4, the permeability was evaluated in a manner similar to that described above using a 1 % solution (5 ml) of ascorbic acid phosphate magnesium salt in PBS. The result is shown in Fig. 5. In the figure, the horizontal axis represents the permeation time and the vertical axis the accumulated amount of ascorbic acid and ascorbic acid phosphate magnesium salt permeated through skin. It is apparent from the figure that the permeation of ascorbic acid and ascorbic acid phosphate magnesium salt is hardly observed in Comparative Example 4. In the case of the composition of Example 6 of the present invention, the permeation rate and accumulated amount of ascorbic acid and ascorbic acid phosphate magnesium salt permeated through skin are remarkably improved.

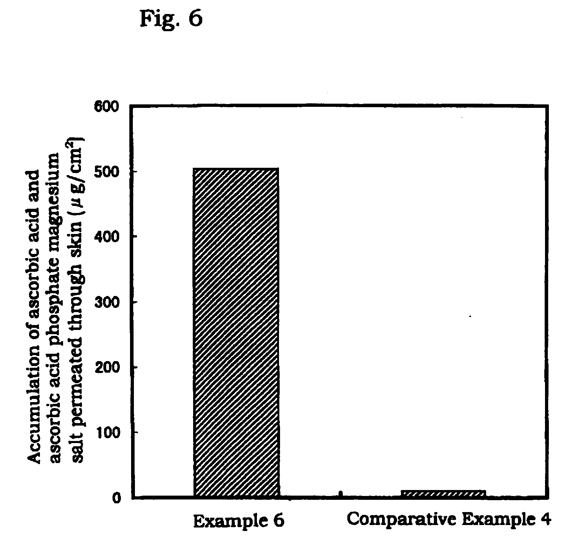

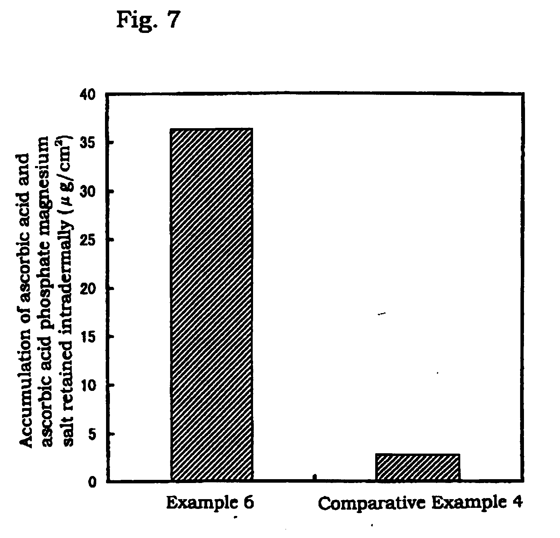

The accumulated amount of ascorbic acid and ascorbic acid phosphate magnesium salt permeated through skin and that retained intradermally were evaluated 24 hours after the start of the cutaneous permeation test *in vitro*. The substances retained intradermally was evaluated by extracting magnesium salt of ascorbate from the test skin with PBS, and measuring by HPLC. The accumulated amount of ascorbic acid and ascorbic acid phosphate magnesium salt permeated through skin after 24 hours is shown in Fig. 6 and that retained intradermally in Fig. 7. From Fig. 6, it can be seen that, in the case of the emulsion composition of Example 6 of the present invention, the amount of ascorbic acid and ascorbic acid phosphate magnesium salt permeated through skin and that retained in the skin greatly increased and are about 50 times and about 10 times, respectively, as much as those shown in Comparative Example 4.

### Experimental Example 3 Observation of Particles with Transmission Electron Microscopy

A portion of the aerosol composition containing liposome and emulsion prepared in Example 6 was air-dried on a sample bench covered with collodion membrane, negatively stained with 1% solution of phosphotungstate (pH 7.0) and observed under a transmission electron microscope (TEM). As shown in Fig. 6. the formation of liposome (particles with bright center), and emulsion (particles with dark center) was confirmed. The figure also shows that the aerosol composition of the present invention contains liposome and emulsion of about 100 to 350 nm diameter.

### INDUSTRIAL APPLICABILITY

The composition of the present invention containing liposome and/or emulsion has excellent characteristics in the cutaneous permeation and intracutaneous retention, and dispersibility at use, and free from problems during a long-term storage such as physical changes due to aggregation or fusion of particles. Further, the composition of the present invention can contain active ingredients together, which cannot co-exist in an ordinary composition for some reasons such as incompatibility due to chemical interactions or the like, because the oil and aqueous phases are kept independently until use. It is generally essential to add a dispersing co-agent into a composition containing liposome and/or emulsion because liposome and emulsion are unstable during storage and the maintenance of the particle size distribution and dispersion state at the time of preparation is almost impossible. On the contrary, such a dispersing co-agent is unnecessary or reducible in the composition of the present invention in which liposome and/or emulsion Is formed at the time of use, and, therefore, it is possible to prevent or reduce the irritant action on skin or mucosa, or hypersensitive reaction by the present composition. Further, the composition of the present invention can be formulated taking no account of the stability of dispersed solution during storage, thereby broadening the freedom of formula design and the utilities of the dispersion.

## Claims

1. A composition containing liposome and/or emulsion, characterized in that it is prepared by colliding the following two solutions at the time of use:
(1) an oil-phase containing a surfactant; and
(2) an aqueous phase, wherein at least one phase contains an active ingredient.

2. A composition containing liposome and emulsion, characterized in that it is prepared by colliding the following two solutions at the time of use:
(1) an oil-phase containing a surfactant and a lipophilic active ingredient; and
(2) an aqueous phase containing a hydrophilic active ingredient(s).

3. A composition containing emulsion, characterized in that it is prepared by colliding the following two solutions at the time of use:
(1) an oil-phase containing a surfactant and a lipophilic active ingredient; and
(2) an aqueous phase.

4. The composition of claim 3 which is non-liposomal.

5. The composition of any one of claims 1 to 4, wherein two solutions are separately kept in a container having a spray mechanism capable of storing the solutions independently, and brought into collision in the spray mechanism at the time of use.

6. The composition of any one of claims 1 to 5, characterized in that the oil-phase comprises one or more solvents selected from the group consisting of alcohols, polyhydric alcohols, fatty acid esters of glycerin, fatty acid esters of sucrose and natural animal or plant oils.

7. The composition of any one of claims 1 to 6, wherein the surfactant is a natural surfactant.

8. A method for preparing a composition containing liposome and/or emulsion, which comprises bringing the following two solutions into collision at the time of use:
(1) an oil-phase containing a surfactant; and
(2) an aqueous phase, wherein at least one phase contains an active ingredient.

9. A method for preparing a composition containing liposome and emulsion, which comprises bringing the following two solutions into collision at the time of use:
(1) an oil-phase containing a surfactant and a lipophilic active ingredient(s); and
(2) an aqueous phase containing a hydrophilic active ingredient.

10. A method for preparing a composition containing emulsion, which comprises bringing the following two solutions into collision at the time of use:
(1) an oil-phase containing a surfactant and a lipophilic active ingredient; and
(2) an aqueous phase.

11. The method of claim 10, wherein the composition is non-liposomal.

12. The method of any one of claims 8 to 11, comprising storing two solutions in a container with a spray mechanism capable of storing the oil- and aqueous phases separately, and bringing the solutions into collision at the time of use.

13. The method of any one of claims 8 to 12, wherein the oil-phase comprises one or more solvents selected from the group consisting of alcohols, polyhydric alcohols, fatty acid esters of glycerin, fatty acid esters of sucrose and natural animal or plant oils.

14. The method of any one of claims 8 to 13, wherein the surfactant is a natural surfactant.
